# EUROPÄISCHE PATENTSCHRIFT

(11) **EP 0 516 983 B1**
(45) Veröffentlichungstag und Bekanntmachung des Hinweises auf die Patenterteilung: **06.08.1997**
(21) Anmeldenummer: 92107544.6
(22) Anmeldetag: 05.05.1992
(51) Int. Cl.: C07D 493/10, C07D 309/18, C07D 301/19

(54) **Verfahren zur Herstellung von 1,6-Dioxaspiro (2.5)octan**
Process for preparation of 1,6-Dioxaspiro (2.5) octans
Procédé pour la préparation des 1,6-Dioxaspiro (2.5) octanes

(30) Priorität: 28.05.1991 DE 4117370
(43) Veröffentlichungstag der Anmeldung: 09.12.1992
(73) Patentinhaber: BASF Aktiengesellschaft, 67063 Ludwigshafen (DE)
(72) Erfinder: Fischer, Martin, Dr., W-6700 Ludwigshafen (DE)

(56) Entgegenhaltungen:
- BULLETIN OF THE ACADEMY OF SCIENCES OF THE USSR,DIVISION OF CHEMICAL SCIENCE Bd. 31, Nr. 9(2), 20. März 1983, GREAT BRITAIN Seiten 1866 - 1873; U.G.IBATULLIN ET AL.: 'Some Reactions of 4-methyl-5,5-dihydropyran and its isomers.'
- CHEMICAL ABSTRACTS, vol. 104, no. 17, 28. April 1986, Columbus, Ohio, US; abstract no. 148683M, GEVORKYAN A.A ET AL.: 'Synthesis and some reactions of 4,4-oxamethylenetetrahydropyran.' Seite 676 ;Spalte 1 ;
- CHEMICAL ABSTRACTS, vol. 113, no. 7, 13. August 1990, Columbus, Ohio, US; abstract no. 58844W, IBATULLIN,U.G. ET AL.: 'Cis-hydroxylation of cyclic unsaturated esters.' Seite 670 ;Spalte 2 ;
- 'Houben-Weyl, Methoden der Organischen Chemie, Band IV/1a, Teil I' 1981 , GEORG THIEME VERLAG , STUTTGART
- 'Houben Weyl, Methoden der Organischen Chemie, Band E13, Organische Peroxo-Verbindungen' 1988 , GEORG THIEME VERLAG , STUTTGART

## Beschreibung

Die vorliegende Erfindung betrifft ein neues Verfahren zur Herstellung von 1,6-Dioxa-spiro[2.5]octan I 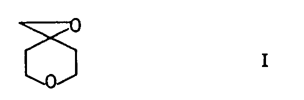 durch Umsetzung von 4-Methylentetrahydropyran II 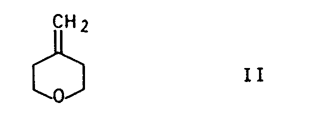 mit einer organischen Peroxoverbindung.

Die Verbindung I ist bekannt und dient als Zwischenprodukt für die Synthese von Tetrahydropyran-4-aldehyd, der seinerseits für die Herstellung von Herbiziden verwendet wird.

Aus Arm. Khim. Zh., 38 (2), 133 (1985), C.A. 104, 148 683 ist die Oxidation von II zu I mit Sauerstoff in Gegenwart von Acetaldehyd oder Benzaldehyd und Nickelacetat bekannt.

In Bull. Acad. Sci USSR, Div. Chem. Sci, 31 (9), S. 1866 (1982) ist die Epoxidation von II zu I mit Perbenzoesäure oder Monoperphthalsäure beschrieben.

Diese bisher bekannten Verfahren vermögen jedoch in verfahrenstechnischer und auch in sicherheitstechnischer Hinsicht nicht zu befriedigen.

Der Erfindung lag deshalb ein technisch verbessertes Verfahren als Aufgabe zugrunde.

Demgemäß wurde ein neues Verfahren zur Herstellung von 1,6-Dioxaspiro[2.5]octan I 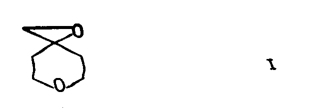 durch Umsetzung von 4-Methylentetrahydropyran II 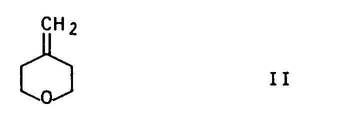 mit einer organischen Peroxoverbindung gefunden, welches dadurch gekennzeichnet ist, daß man II mit einem organischen Hydroperoxid in Gegenwart eines Carboxylates, Alkoholates oder einer Komplexverbindung des Titans, Vanadiums, Molybdäns oder Wolframs als Katalysator umsetzt.

Das erfindungsgemäße Verfahren läßt sich wie folgt veranschaulichen: 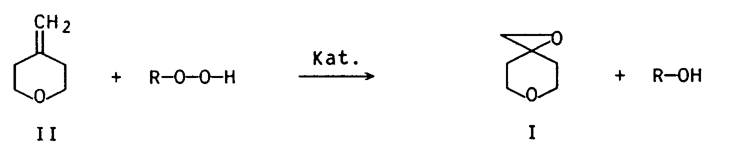 R = C-organischer Rest

Die Ausgangsverbindung 4-Methylentetrahydropyran II ist bekannt oder nach bekannten Methoden erhältlich, z.B. durch Umsetzung von 3-Methylenpentan-1,5-diol mit einem Sulfonsäurehalogenid in Gegenwart einer wäßrigen Mineralbase (DE-A 39 26 170).

Als organische Hydroperoxide kommen vornehmlich solche der Formel III in Betracht: 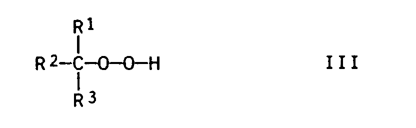 in der
- R¹ und R²: Alkylgruppen bezeichnen, die zu einem 5- oder 6-gliedrigen Ring verbunden sein können und teilweise oder vollständig mit Halogen wie Chlor oder vorzugsweise Fluor substituiert sein können und
- R³: Wasserstoff, eine unsubstituierte oder substituierte Phenylgruppe, eine Alkyl- oder Halogenalkylgruppe bedeutet.

Unter den definitionsgemäßen Hydroperoxiden III werden aus wirtschaftlichen Gründen solche bevorzugt, in denen die Reste R¹ und R² folgende Bedeutung haben:
- C₁-C₄-Alkyl, vorzugsweise C₁-C₂-Alkyl, darunter vorzugsweise Methyl;
- C₅- und C₆-Cycloalkyl wie Cyclopentyl und vor allem Cyclohexyl;
- Halogenalkyl wie Chloralkyl und vorzugsweise Fluoralkyl.

Als Rest R³ eignet sich vorzugsweise:
- Wasserstoff;
- Phenyl;
- C₁-C₄-Alkyl, vorzugsweise C₁-C₂-Alkyl, darunter vorzugsweise Methyl;
- Halogenalkyl wie Chloralkyl und vorzugsweise Fluoralkyl.

Als Substituenten im Phenylrest kommen vorzugsweise Methyl und Chlor in Betracht.

Bevorzugte Hydroperoxide sind
- Cumolhydroperoxid,
- t-Amylhydroperoxid,
- 1-Phenylethylhydroperoxid,
- 2-Phenylethylhydroperoxid,
- Cyclohexanhydroperoxid.

Besonders bevorzugt ist t-Butylhydroperoxid. Das Molverhältnis von II zum Hydroperoxid beträgt zweckmäßigerweise 1:1 bis 10:1, bevorzugt 1,5:1 bis 3:1.

Von den im erfindungsgemäßen Verfahren einzusetzenden Katalysatoren verwendet man vorzugsweise die des Wolframs und Vanadiums sowie vor allem Molybdänkatalysatoren. Man kann die Katalysatoren in Form von Carboxylaten, Alkoholaten oder in Form von Komplexverbindungen einsetzen.

Bevorzugt sind Acetylacetonate, o-Hydroxybenzoate und Carbonyle, besonders bevorzugt Carboxylate und ganz besonders bevorzugt Alkoholate.

Vorzugsweise verwendet man Wolfram-, Vanadium- und Molybdänacetylacetonat und -o-Hydroxybenzoat, Molybdän- und Wolframhexacarbonyl, Carboxylate von aliphatischen Carbonsäuren wie Naphthen- oder 2-Ethylhexansäure und vor allem Alkoholate von aliphatischen Mono- oder Diolen wie Isopropanol, 2-Ethylhexanol oder Propylendiglykol und Weinsäuredialkylester.

Man kann die Katalysatoren in Form von Trägerkatalysatoren auf Trägern wie Aluminumoxid, Siliciumoxid, Titanoxid, Zinkoxid, Lanthanoxid, Zirkonoxid, Bariumsulfat, Aluminiumsilikat, Aktivkohle oder Ionenaustauscherharzen verwenden.

Die Trägerkatalysatoren haben vorzugsweise einen Gehalt an aktivem Metall von 0,1 bis 5, besonders 0,1 bis 2 Gew.-%, bezogen auf die Gesamtmenge von Träger und katalytisch aktiven Metallen, berechnet als Metall.

Die Katalysatoren können wahlweise als 2- bis 4-mm-Stränge oder als Tabletten mit 3 bis 5 mm Durchmesser, als Splitt mit Teilchengrößen von 0,05 bis 1 mm, vorzugsweise 0,1 bis 0,5 mm als größten Durchmesser oder als Pulver mit Teilchengrößen von 0,005 bis 0,5 mm eingesetzt werden.

Man kann die Reaktion diskontinuierlich oder kontinuierlich vornehmen. So kommt eine Festbettreaktion in Sumpf- oder Rieselfahrweise in der Flüssigphase in Betracht.

Die Menge der Katalysatoren, die man auch in ihren Mischungen einsetzen kann, wird bei diskontinuierlichen Verfahren vorzugsweise so bemessen, daß man 0,01 bis 1 mol-%, vorzugsweise 0,05 bis 0,5 mol-% des Metalls zur Verfügung stellt, bezogen auf die Menge der Verbindung II.

Beim kontinuierlichen Verfahren beträgt die Katalysatorbelastung im allgemeinen 10 g bis 200 g der Verbindung II pro kg Katalysator und Stunde.

In der Regel führt man die Reaktion bei 40 bis 130°C, vorzugsweise bei 70 bis 100°C durch.

Zweckmäßigerweise wird die Umsetzung bei Normaldruck vorgenommen, jedoch kann man auch bei vermindertem oder leicht erhöhtem Druck arbeiten, also etwa im Bereich von 10 mbar bis 20 bar.

Die Reaktionszeiten betragen normalerweise 1 bis 12, meistens 1 bis 6 Stunden.

Es kann zweckmäßig sein, die Umsetzung unter Mitverwendung von Lösungsmitteln durchzuführen. Hierbei eignen sich aromatische Kohlenwasserstoffe wie Toluol, Xylol und Cumol, aliphatische Kohlenwasserstoffe wie Heptan und Hexan, cycloaliphatische Kohlenwasserstoffe wie Cyclohexan oder halogenierte Kohlenwasserstoffe wie Chlorbenzol, Dichlorbenzol und Dichlorethan. Weiterhin kommen t-Butanol und Aceton in Betracht.

Außerdem eignen sich die Flüssigphasenreaktion mit löslichen Katalysatoren oder suspendierten Trägerkatalysatoren. Vorzugsweise arbeitet man diskontinuierlich.

Eine wirtschaftlich sehr vorteilhafte Ausführungsform des erfindungsgemäßen Verfahrens besteht in der Durchführung mit gelösten Homogenkatalysatoren. 4-Methylentetrahydropyran II wird in Gegenwart eines gelösten Homogenkatalysators erhitzt und mit einem organischen Hydroperoxid, gegebenenfalls im Gemisch mit einem Lösungsmittel, nach Maßgabe der Reaktivität des Hydroperoxides im Zulaufverfahren versetzt.

Die Aufarbeitung des Reaktionsgemisches auf das Verfahrensprodukt I erfolgt in an sich bekannter Weise, und zwar in der Regel durch Destillation, gewünschtenfalls nach Zerstörung von Restmengen Hydroperoxid.

Das nach dem erfindungsgemäßen Verfahren auf einfache und wirtschaftliche Weise erhältliche 1,6-Dioxaspiro[2.5]octan I dient als Zwischenprodukt für die Synthese von Tetrahydropyran-4-aldehyd, der seinerseits für die Herstellung von Herbiziden verwendet wird (DE-A 31 21 355).

### Beispiel

### Herstellung von 1,6-Dioxaspiro[2.5]octan

588 g 4-Methylentetrahydropyran und 9,9 g Molybdänethylhexanoat (Mo-Gehalt: 6,8 Gew.-%) wurden auf 80°C erhitzt und innerhalb von 3 Stunden mit 780 g eines 1:1-Gemisches aus t-Butanol und t-Butylhydroperoxid versetzt.

Danach wurde zur Vervollständigung der Reaktion für die Dauer von drei weiteren Stunden bei 80°C gerührt.

Nach Abkühlung lieferte die übliche Aufarbeitung durch Destillation bei 58°C/24 mbar 85 % 1,6 Dioxaspiro[2.5]octan.

## Patentansprüche

1. Verfahren zur Herstellung von 1,6-Dioxaspiro[2.5]octan I 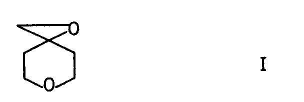 durch Umsetzung von 4-Methylentetrahydropyran II 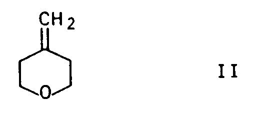 mit einer organischen Peroxoverbindung, dadurch gekennzeichnet, daß man II mit einem organischen Hydroperoxid in Gegenwart eines Carboxylates, Alkoholates oder einer Komplexverbindung des Titans, Vanadiums, Molybdäns oder Wolframs als Katalysator umsetzt.

2. Verfahren nach Anspruch 1, dadurch gekennzeichnet, daß man als Hydroperoxid t-Butylhydroperoxid verwendet.

3. Verfahren nach Anspruch 1 oder 2, dadurch gekennzeichnet, daß man die Reaktion in Gegenwart von gelösten Homogenkatalysatoren vornimmt.

## Claims

1. A process for preparing 1,6-dioxaspiro[2.5]octane I 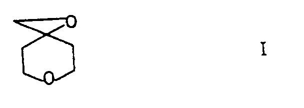 by reacting 4-methylenetetrahydropyran II 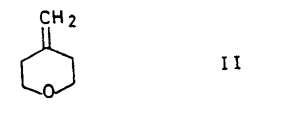 with an organic peroxo compound, characterized in that II is reacted with an organic hydroperoxide in the presence of a carboxylate, alkoxide or complex of titanium, vanadium, molybdenum or tungsten as catalyst.

2. A process as claimed in claim 1, characterized in that the hydroperoxide used is t-butyl hydroperoxide.

3. A process as claimed in claim 1 or 2, characterized in that the reaction is carried out in the presence of dissolved homogeneous catalysts.

## Revendications

1. Procédé de préparation du 1,6-dioxaspiro[2.5]octane I 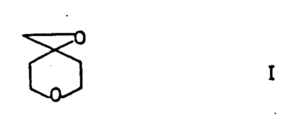 par réaction du 4-méthylènetétrahydropyranne II 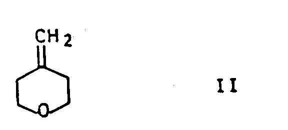 avec un composé organique en peroxo, caractérisé par le fait que l'on fait réagir le composé II avec un hydroperoxyde organique en présence d'un carboxylate, d'un alcoolate ou d'un complexe du titane, du vanadium, du molybdène ou du tungstène qui sert de catalyseur.

2. Procédé selon la revendication 1, caractérisé par le fait que l'hydroperoxyde utilisé est l'hydroperoxyde de tert-butyle.

3. Procédé selon la revendication 1 ou 2, caractérisé par le fait que l'on exécute la réaction en présence de catalyseurs homogènes dissous.
